# EUROPEAN PATENT APPLICATION

(11) **EP 1 442 763 A1**
(43) Date of publication of application: **04.08.2004**
(21) Application number: 03250511.7
(22) Date of filing: 28.01.2003
(51) Int. Cl.: A61M 5/32

(54) **Safety syringe with needle retracted by vacuum**

(71) Applicant: Yang, Chung-Yu, Taipei City (TW)
(72) Inventor: Yang, Chung-Yu, Taipei City, Taiwan ROC (TW)
(74) Representative: Nicholls, Michael John

(57) **Abstract**

A safety syringe includes a flexible holder-supporting seat (24, 24') that is sleeved around and that clamps a needle holder (30, 30') within a front end portion of a syringe barrel (2, 2'). A flexible sealing member (5, 5') seals an open front end of a plunger (4, 4') so as to define a vacuum chamber (40A) in the plunger (4, 4'). When the plunger (4, 4') moves within the barrel (2, 2') to a front limit position, a holder-retaining front portion (51, 51') of the sealing member (5, 5') engages and retains the needle holder (30, 30') thereon, and the plunger (4, 4') pushes the holder-supporting seat (24, 24') to separate from the needle holder (30, 30') such that the sealing member (5, 5') and the needle holder (30, 30') move rearward within the syringe barrel (2, 2') due to negative pressure produced in the plunger (4, 4'), thereby retracting a needle (31, 31') into the syringe barrel (2, 2').

## Description

This invention relates to a medical syringe, more particularly to a safety syringe with an automatically retractable needle.

After using a disposable medical syringe, the user may be injured by accidental puncture of a needle of the syringe. PCT Application No. 000287 and U.S. Patent Nos. 5,395,337 and 6,077,245 disclose safety syringes that are provided with retractable needles, which can be withdrawn into syringe barrels or plungers by means of a negative pressure or a spring force to prevent accidental puncture. However, the structures of the aforesaid conventional safety syringes are too complex to fabricate at relatively low costs.

The object of this invention is to provide a safety syringe, which includes a retractable needle and which has a relatively simple structure.

According to this invention, a safety syringe includes a flexible holder-supporting seat that clamps a needle holder within a front end portion of a syringe barrel. A flexible sealing member seals an open front end of a plunger so as to define a vacuum chamber in the plunger. When the plunger moves within the syringe barrel to a front limit position, a holder-retaining front portion of the sealing member engages and retains the needle holder thereon, and the plunger pushes the holder-supporting seat to separate from the needle holder such that the sealing member and the needle holder move rearward within the syringe barrel due to negative pressure produced within the plunger, thereby retracting a needle into the syringe barrel.

These and other features and advantages of this invention will become,apparent in the following detailed description of the preferred embodiments of this invention, with reference to the accompanying drawings, in which:
Fig. 1 is an exploded perspective view of a first preferred embodiment of a safety syringe according to this invention;
Fig. 2 is a sectional view of the first preferred embodiment;
Fig. 3 is a sectional view of a syringe barrel and a needle unit of the first preferred embodiment;
Fig. 4 is a sectional view of a plunger and a sealing member of the first preferred embodiment;
Fig. 5 illustrates how the sealing member is moved together with the plunger when the plunger is pulled rearward relative to the syringe barrel;
Fig. 6 illustrates how a holder-retaining front portion of the sealing member of the first preferred embodiment engages a needle holder;
Fig. 7 illustrates how a holder-supporting seat of the first preferred embodiment is pushed by the plunger to separate from the needle holder;
Fig. 8 illustrates how the needle unit of the first preferred embodiment is retracted into the plunger;
Fig. 9 is a sectional view of a second preferred embodiment of a safety syringe according to this invention;
Fig. 10 illustrates how a holder-retaining front portion of a sealing member of the second preferred embodiment engages a needle holder;
Fig. 11 illustrates how a holder-supporting seat of the second preferred embodiment is pushed by a plunger to separate from the needle holder; and
Fig. 12 illustrates how a needle unit of the second preferred embodiment is retracted into the plunger.

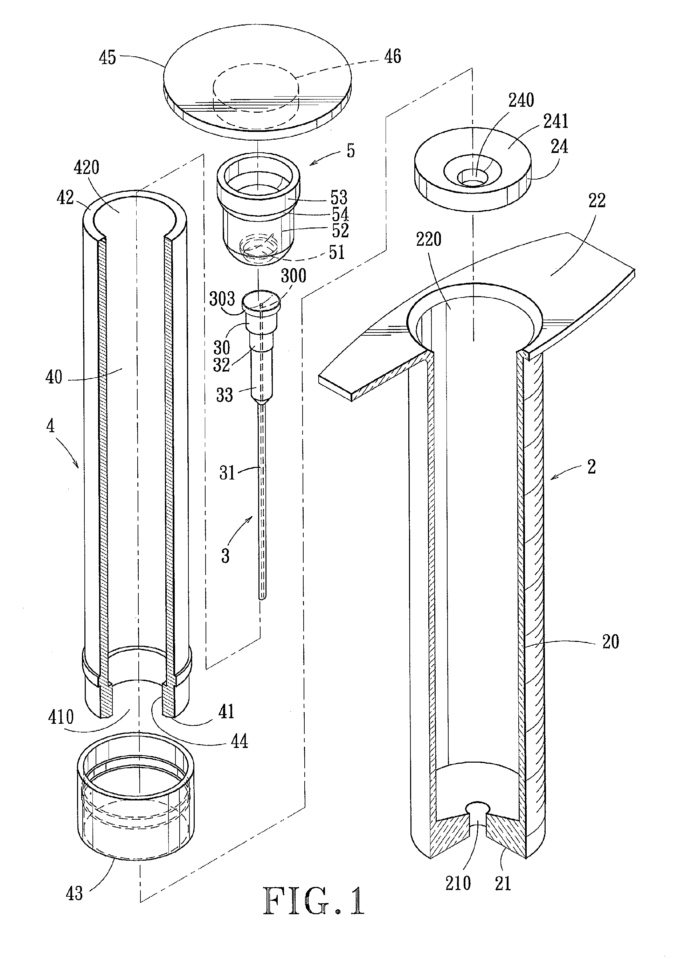

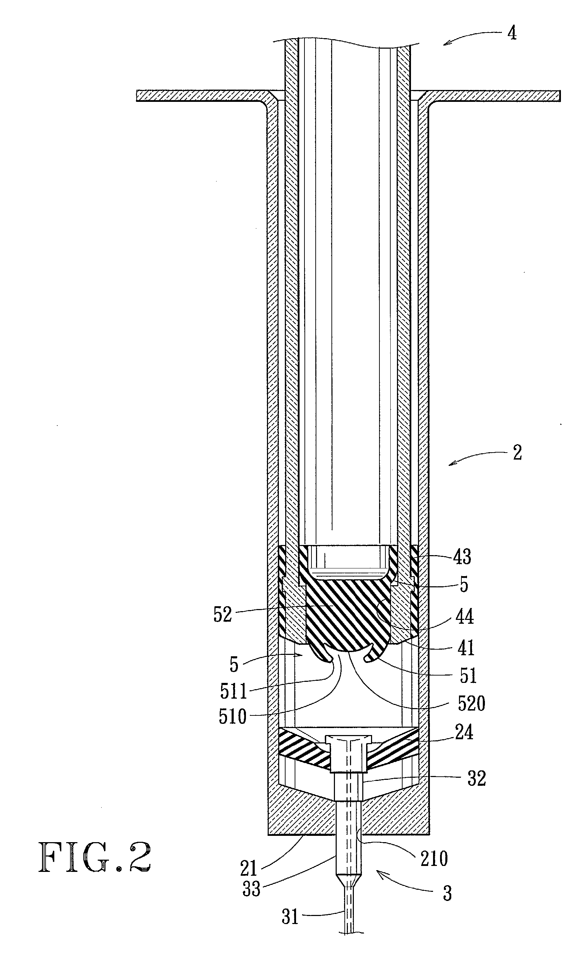

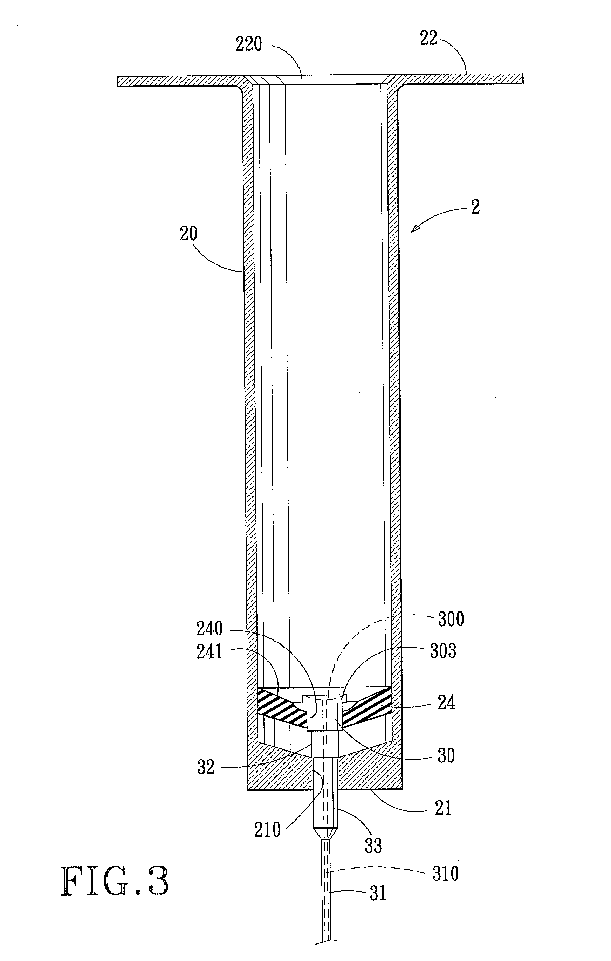

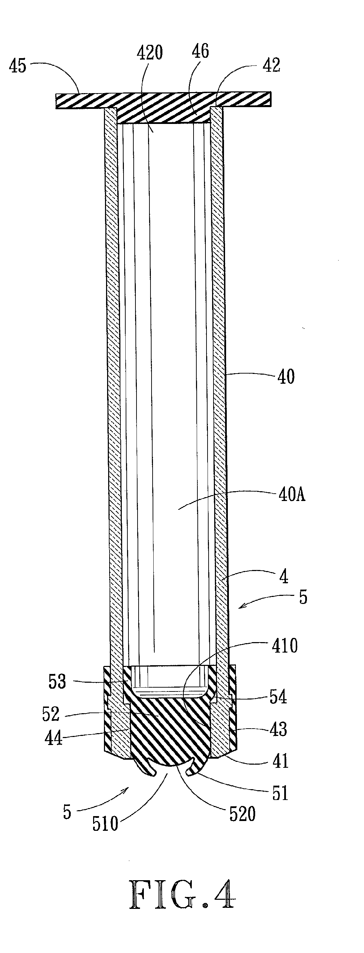

Referring to Figs. 1, 2, 3, and 4, a first preferred embodiment of a safety syringe according to this invention is shown to include a syringe barrel 2, a needle unit 3, a plunger 4, and a flexible sealing member 5. The needle unit 3 has a needle holder 30, a needle 31, a spacer portion 32 having a rear end that is formed integrally with a front end of the needle holder 30, and a connecting portion 33 having a front end that is formed integrally with a rear end of the needle 31, and a rear end that is formed integrally with a front end of the spacer portion 32.

The syringe barrel 2 includes a barrel body 20, a front end wall 21 that is formed integrally with a front end of the barrel body 20 and that has a front opening 210, a rear end wall 22 that is formed integrally with a rear end of the barrel body 20 and that has a rear opening 220, and an annular flexible holder-supporting seat 24 with frustoconical front and rear end surfaces 241, each of which has a diameter that increases rearward. The holder-supporting seat 24 is sleeved on the needle holder 30, and has a central hole 240 formed therethrough, an outer periphery that is in frictional contact with an inner surface of the syringe barrel 2 in such a manner that a liquid-tight seal is established therebetween, and an inner periphery that is in frictional contact with an outer surface of the needle holder 30 of the needle unit 3 in such a manner that a liquid-tight seal is established therebetween.

The needle unit 3 is inserted into the syringe barrel 2 through the rear opening 220. The connecting portion 33 of the needle unit 3 extends through the front opening 210 in the syringe barrel 2. The needle 31 is exposed outwardly from the front opening 210. The spacer portion 32 is sized to prevent movement of the spacer portion 32 into the front opening 210. As such, the needle holder 30 is clamped within the central hole 240 in the holder-supporting seat 24 at a position that is spaced apart from the front end wall 21 at a predetermined distance. The needle holder 30 has a rear end that is formed with an outward flange 303 which extends integrally, radially, and outwardly therefrom and which is sized to prevent forward movement of the outward flange 303 into the central hole 240 in the holder-supporting seat 24.

The plunger 4 is disposed movably within the syringe barrel 2, and includes a plunger body 40 that has an open front end 41 which is formed with a front opening 410 so that the sealing member 5 can be placed into the plunger body 40 and can extend therethrough during assembly, and an open rear end 42 that is formed with a rear opening 420. A unitary rear end wall 45 has a circular projection 46 extending forwardly therefrom and press-fitted within the rear opening 420 in the plunger body 40. When the plunger 4 is to be evacuated, the rear end wall 45 can be removed from the plunger body 40 for evacuation of air from the plunger 4. A rubber seal ring 43 is disposed between the plunger 4 and the syringe barrel 2 so as to establish a liquid-tight seal therebetween. The plunger body 40 has a front end that is formed with an inward flange 44 extending integrally, radially, and inwardly therefrom.

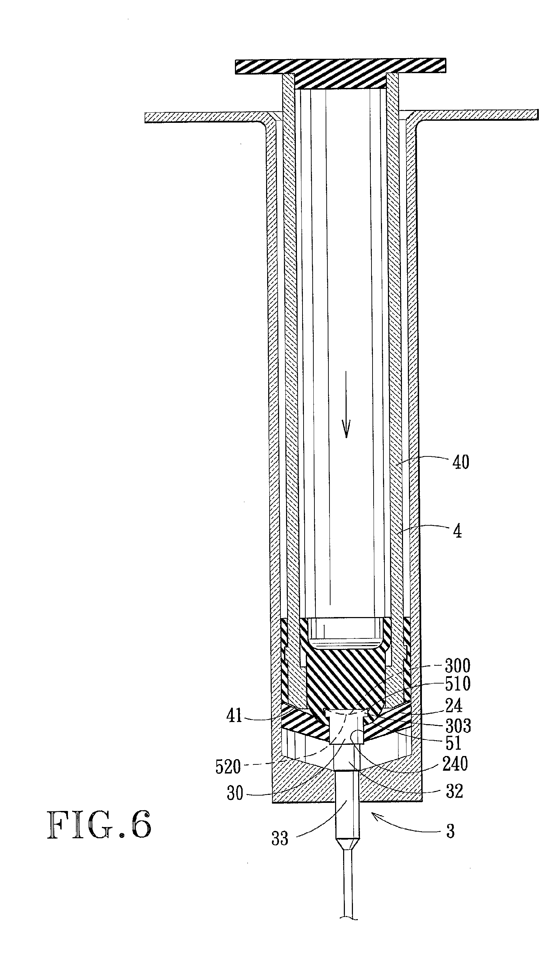

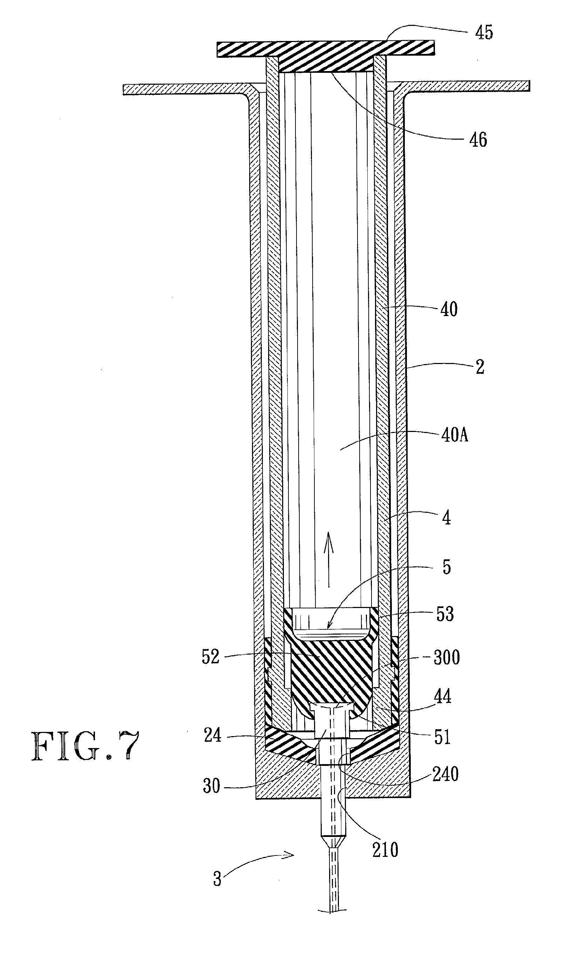

The sealing member 5 is disposed movably within the front end portion of the plunger 4 so as to define a vacuum chamber (40A) in the plunger 4 between the sealing member 5 and the rear wall 45. The sealing member 5 is unitary, is made of rubber, and includes a holder-retaining front portion 51 disposed behind and spaced apart from the needle holder 30, a sealing rear portion 52 for closing the front opening 410 in the plunger 4, a rear end skirt portion 53, and a shoulder 54 defined between the sealing rear portion 52 and the skirt portion 53. The inward flange 44 of the plunger 4 is sleeved around and clamps the sealing rear portion 52 of the sealing member 5 in the plunger 4 so as to prevent movement of the sealing member 5 relative to the plunger 4, thereby permitting synchronous rearward movement of the sealing member 5 and the plunger 4 when the plunger 4 is pulled rearward relative to the syringe barrel 2. The holder-retaining front portion 51 is shaped as an annular flange that extends forward and inwardly to define a blind hole 510 and that has a rounded front end edge 511 for guiding movement of the outward flange 303 of the needle holder 30 into the blind hole 510 when the outward flange 303 moves into the blind hole 510. The blind hole 510 has a front end that has a diameter which is slightly smaller than that of the outward flange 303 so as to confine the outward flange 303 within the blind hole 510 when the outward flange 303 moves into the blind hole 510, as shown in Figs. 6 and 7. The holder-retaining front portion 51 is formed with a curved projection 520 that is located within the blind hole 510 and that is movable within the syringe barrel 2 to seal the rear opening 300 in the needle holder 30 when the outward flange 303 engages the blind hole 510.

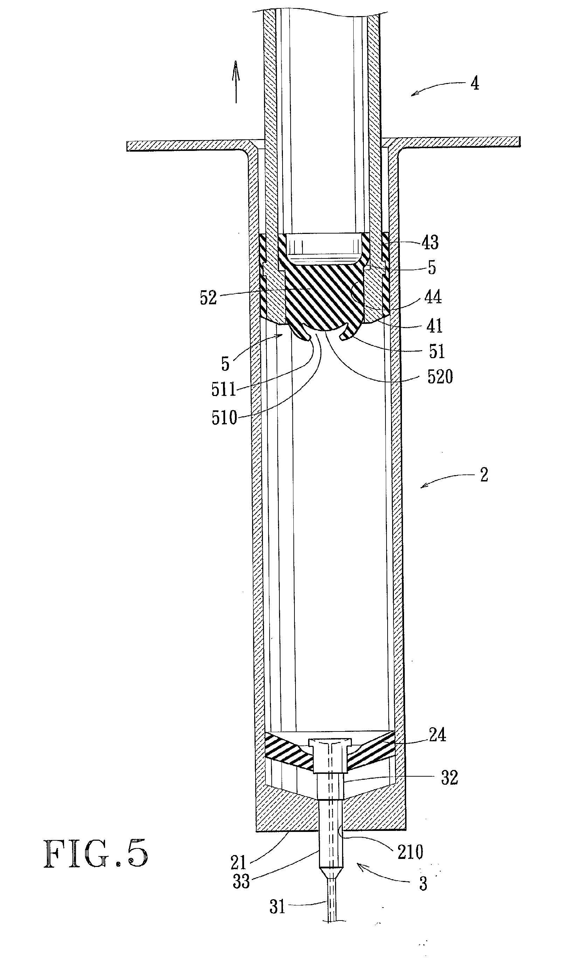

Referring to Fig. 5, when the plunger 4 is pulled rearward relative to the syringe barrel 2 in order to draw a medical liquid, blood, or body liquid from a subject, a rear edge of the inward flange 44 of the plunger 4 engages the shoulder 54 of the sealing member 5 so as to permit synchronous movement of the sealing member 5 and the plunger 4.

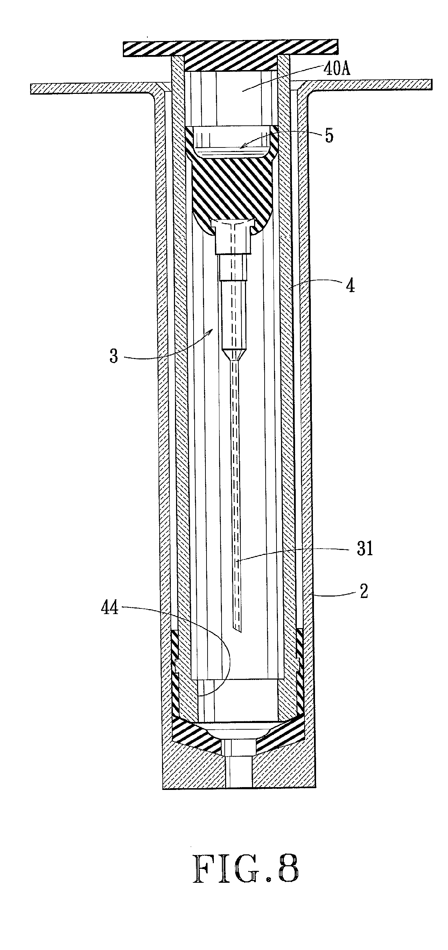

When it is desired to inject a medical liquid into a body, the plunger 4 is moved to a position shown in Fig. 6, where the outward flange 303 of the needle holder 30 engages the blind hole 510 in the sealing member 5, and where a front end of the plunger body 40 comes into contact with the holder-supporting seat 24. Thereafter, the sealing member 5 is blocked by the needle holder 30 from further forward movement within the syringe barrel 2. The plunger 4 continues to move forward within the syringe barrel 2 to a front limit position shown in Fig. 7, where the holder-supporting seat 24 is pushed by the plunger 4 to separate from the needle holder 30 and where the rear sealing rear portion 52 of the sealing member 5 is released from the inward flange 44 of the plunger 4. As such, an assembly of the sealing member 5 and the needle unit 3 will move rearward within the plunger 4 due to negative pressure produced within the evacuated plunger 4 until it moves to a retracted position shown in Fig. 8, where the needle unit 3 is concealed within the plunger 4.

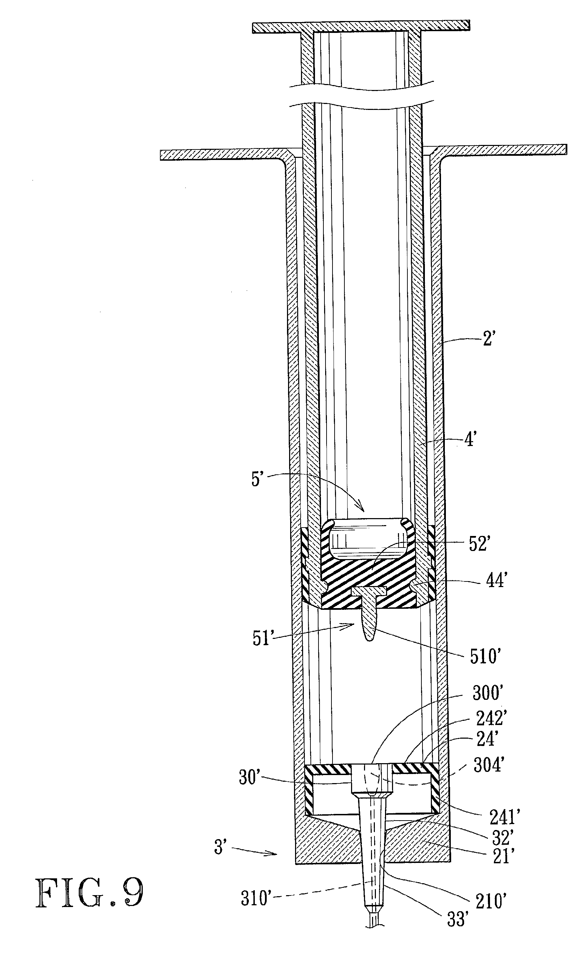

Referring to Fig. 9, a second preferred embodiment of a safety syringe according to this invention is shown to include a syringe barrel 2', a needle unit 3', a plunger 4', and a sealing member 5'. The differences between the first and second preferred embodiments are described in the succeeding paragraph.

The syringe barrel 2' includes a flexible holder-supporting seat 24' constructed as a hollow cylinder that has a surrounding wall 241' in frictional contact with an inner surface of the syringe barrel 2' , and a ring-shaped rear end wall 242' sleeved around and clamping a rear end of a needle holder 30' of the needle unit 3' therein. The central bore 310' in the needle unit 3' has an enlarged rear bore portion 304' that has a greatest diameter slightly greater than that of a rear opening 300' in the needle holder 30'. A holder-retaining front portion 51' of the sealing member 5' includes a fixed flexible insert member 510' that is movable within the syringe barrel 2' to engage fittingly the enlarged rear bore portion 304' of the central bore 310' in the needle unit 3' so as to retain the needle holder 30' on the sealing member 5'. The plunger 4' has a front end portion with an inward flange 44' that is configured as an annular rib which presses against a sealing rear portion 52' of the sealing member 5'. As such, the sealing member 5' can be removed forcibly from the plunger 4' for evacuation of air from the plunger 4'. The syringe barrel 2' has a front end wall 21' that is formed with a tapered front opening 210'. The needle unit 3' includes the needle holder 30', from which an outward flange similar to that in the previous embodiment is omitted, a needle 31', a spacer portion 32', and a connecting portion 33'. An assembly of the spacer portion 32' and the connecting portion 33' is shaped as a truncated cone. The connecting portion 33' extends through the front opening 210' in the syringe barrel 2'. The spacer portion 32' is sized to prevent movement into the front opening 210'.

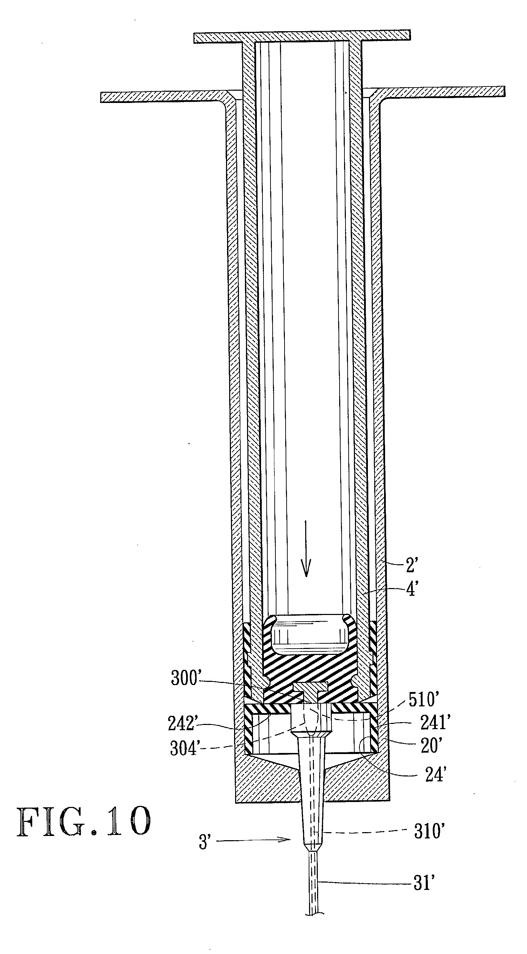

The plunger 4' is movable forward within the syringe barrel 2' to a position shown in Fig. 10, where the insert member 510' engages the enlarged bore portion 304' of the central bore 310' in the needle unit 3' so as to prevent further forward movement of the sealing member 5' within the syringe barrel 2' and where a front end of the plunger 4' comes into contact with the rear end wall 24'.

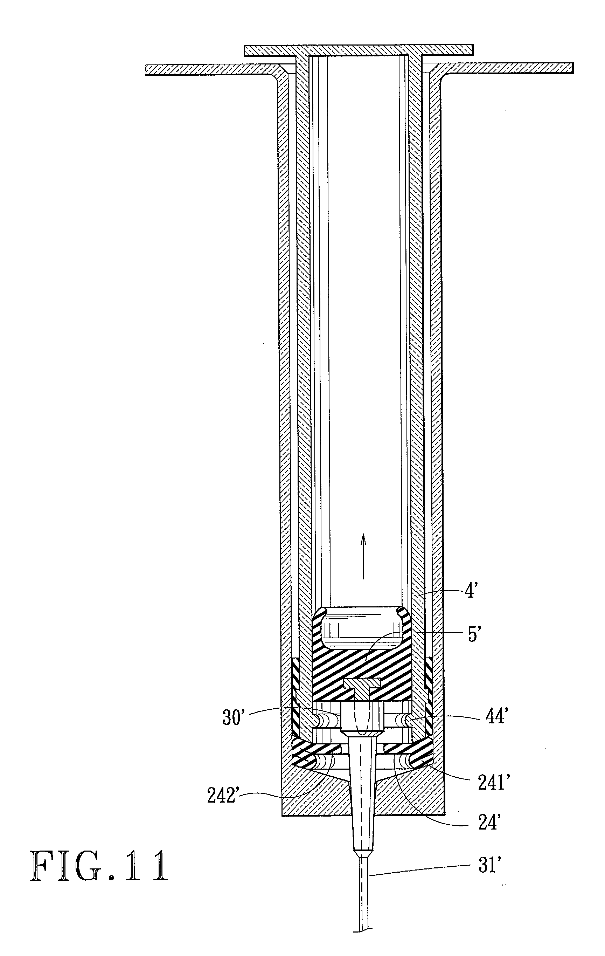

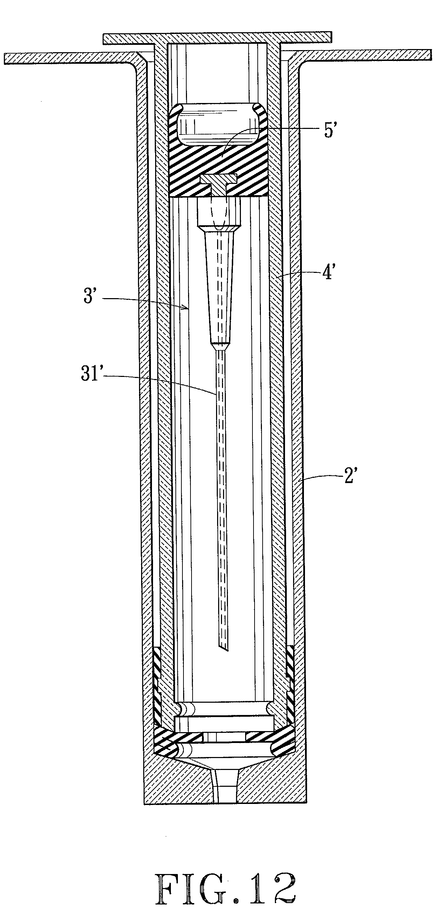

The plunger 4 will continue to move forward within the syringe barrel 2' to a front limit position shown in Fig. 11, where the inward flange 44' separates from the sealing member 5' and where the surrounding wall 241' of the holder-supporting seat 24' deforms to permit forward removal of the rear end wall 242' from the needle holder 30'. As such, the needle unit 3' can also be drawn into the plunger 4' due to negative pressure produced in the plunger 4', as shown in Fig. 12.

## Claims

1. A safety syringe including:
a syringe barrel (2, 2') having a front end wall (21, 21') with a front opening (210, 210'), and a rear end wall (22) with a rear opening (220);
a plunger (4, 4') disposed movably within the syringe barrel (2, 2') and having a front end (41) and a rear end (42) that extends from the rear opening (220) in the syringe barrel (2, 2'); and
a needle unit (3, 3') extending through the front opening (210, 210') in the syringe barrel (2, 2') and including a central bore (310, 310') formed therethrough, a needle holder (30, 30') that is formed with a rear opening (300, 300'), and a needle (31) that is connected fixedly to the needle holder (30, 30') at a rear end and that is exposed outwardly from the front opening (210, 210') in the syringe barrel (2, 2');
**characterized by**:
the syringe barrel (2, 2') including an annular flexible holder-supporting seat (24, 24') disposed movably within the syringe barrel (2, 2') and sleeved around the needle holder (30, 30'), the holder-supporting seat (24, 24') having a central hole (240, 240') formed therethrough, an outer periphery that is in frictional contact with an inner surface of the syringe barrel (2, 2') in such a manner that a liquid-tight seal is established therebetween, and an inner periphery that is in frictional contact with an outer surface of the needle holder (30, 30') in such a manner that a liquid-tight seal is established therebetween;
said plunger (4, 4') having an open front end (41) that is formed with a front opening (410), a rear end wall (45) , and a front end portion that is formed with an inward flange (44, 44') extending integrally, radially, and inwardly therefrom;
a flexible sealing member (5, 5') being disposed movably within the front end portion of the plunger (4, 4') so as to define a vacuum chamber in the plunger (4, 4') between the sealing member (5, 5') and the rear end wall (45), the inward flange (44, 44') being sleeved around and clamping the sealing member (5, 5') in the plunger (4, 4') so as to prevent movement of the sealing member (5, 5') relative to the plunger (4, 4'), thereby permitting synchronous rearward movement of the sealing member (5, 5') and the plunger (4, 4') when the plunger (4, 4') is pulled rearward relative to the syringe barrel (2, 2'), the sealing member (5, 5') having a holder-retaining front portion (51, 51') that is disposed behind and that is spaced apart from the needle holder (30, 30'), and a sealing rear portion (52, 52') for closing the front opening (410) in the plunger (4, 4') ;
said needle unit (3, 3') having a spacer portion (32, 32') that is formed integrally between the needle holder (30, 30') and the needle (31, 31') and that is sized to prevent movement of the spacer portion (32, 32' ) into the front opening (210, 210')) in the syringe barrel (2, 2') such that the needle holder (30, 30') is clamped within the holder-supporting seat (24, 24') at a position that is spaced apart from the front end wall (21, 21') by a predetermined distance;
said holder-retaining front portion (51, 51') of the sealing member (5, 5' ) moving forward to retain the needle holder (30, 30') thereon when the plunger (4, 4') is moved forward within the syringe barrel (2, 2') to push the holder-supporting seat (24, 24') forward such that the holder-supporting seat (24, 24') separates from the needle holder (30, 30') so as to permit automatic rearward movement of an assembly of the sealing member (5, 5' ) and the needle unit (3, 3') within the syringe barrel (2, 2') due to negative pressure produced within the plunger (4, 4'), thereby retracting the needle (31, 31') into the syringe barrel (2, 2').

2. The safety syringe as claimed in Claim 1, **characterized in that** the needle unit (3) is formed with an outward flange (303) that extends integrally, radially, and outwardly from a rear end of the needle holder (30) and that is sized to prevent forward movement of the outward flange (303) into the central hole (240) in the holder-supporting seat (24), the holder-retaining front portion (51) of the sealing member (5) being shaped as an annular flange that extends forwardly and inwardly to define a blind hole (510) and that has a rounded front end (511) for guiding movement of the outward flange (303) of the needle holder (30) into the blind hole (510) , the blind hole (510) including a front end that has a diameter which is slightly smaller than that of the outward flange (303) of the needle unit (3) so as to confine the outward flange (303) within the blind hole (510) when the outward flange (303) moves into the blind hole (510).

3. The safety syringe as claimed in Claim 2, further **characterized in that** the holder-retaining front portion (51) of the sealing member (5) is formed with a curved projection (520) that is located within the blind hole (510) and that is movable within the syringe barrel (2) to seal the rear opening (300) in the needle holder (30) when the outward flange (303) engages the blind hole (510).

4. The safety syringe as claimed in Claim 1, **characterized in that** the plunger (4) includes a plunger body (40) that has an open rear end (420) so that the sealing member (5) can be placed into the plunger body (40) and can extend therethrough during assembly, the rear end wall (45) of the plunger (4) being unitary and having a circular proj ection (46) that extends forwardly therefrom and that is press-fitted within the rear end (420) of the plunger body (40) so as to be removable from the plunger body (40) .

5. The safety syringe as claimed in Claim 1, **characterized in that** the holder-supporting seat (24' ) is constructed as a hollow cylinder that has a surrounding wall (241') in frictional contact with the inner surface of the syringe barrel (2'), and a ring-shaped rear end wall (242') sleeved around and clamping a rear end of the needle holder (30') therein, the central bore (310') in the needle unit (3') having an enlarged rear bore portion (304') that has a greatest diameter slightly greater than that of the rear opening (300') in the needle holder (30'), the holder-retaining front portion (51') of the sealing member (5') including a fixed flexible insert member (510') that is movable within the syringe barrel (2') to engage fittingly the enlarged rear bore portion (304') of the central bore (310') so as to retain the needle holder (30') on the sealing member (5').
